# EUROPEAN PATENT SPECIFICATION

(11) **EP 2 934 694 B1**
(45) Date of publication and mention of the grant of the patent: **28.06.2023**
(21) Application number: 13865076.7
(22) Date of filing: 20.12.2013
(51) Int. Cl.: A61K 8/36, A61K 8/365, A61K 8/44, A61K 8/46, A61K 8/55, A61Q 11/00, A61P 1/02

(54) **ANTI-PLAQUE ORAL COMPOSITIONS**
ORALE ZUSAMMENSETZUNGEN GEGEN PLAQUE
COMPOSITIONS ORALES ANTI-PLAQUE DENTAIRE

(30) Priority: 20.12.2012 US 201261740391 P
(43) Date of publication of application: 28.10.2015
(73) Proprietor: Bhushan, Rajiv, Mountain View, California 94041 (US); Gin, Jerry B., Sunnyvale, California 94807 (US); Goswamy, Amit, Los Gatos, California 95030 (US)
(72) Inventor: Bhushan, Rajiv, Mountain View, California 94041 (US); Gin, Jerry B., Sunnyvale, California 94807 (US); Goswamy, Amit, Los Gatos, California 95030 (US)
(74) Representative: J A Kemp LLP
(86) International application number: PCT/US2013/077330
(87) International publication number: WO 2014/100775

(56) References cited:
- WO-A1-2005/094765
- WO-A1-2013/166459
- WO-A2-2012/146832
- US-A- 2 975 102
- US-A- 3 819 826
- US-A1- 2006 134 020
- US-A1- 2006 147 392
- US-A1- 2010 035 992
- US-B1- 6 193 958
- US-B1- 6 645 472
- DATABASE WPI Thomson Scientific, London, GB; AN 2007-170738 XP002758810, & SE 529 208 C2 (VIKNER S) 29 May 2007 (2007-05-29)
- FRUEH-VON ARX ET AL: "Experimentelle Prilfung des Diffusionsvermogens von Dimethylsulfoxyd in menschlichen Zahnen", SCHWEIZERISCHE MONATSSCHRIFT FUER ZAHNHEILKUNDE, BERICHTHAUS, ZUERICH, CH, vol. 79, no. 11, 1 January 1969 (1969-01-01), pages 1247-1261, XP008158551, ISSN: 0036-7702

## Description

### CROSS-REFERENCE TO RELATED APPLICATIONS

This international application filed under the Paris Convention Treaty (PCT) claims priority to U.S. Provisional Patent Application Serial No. 61/740,391 filed December 20, 2012 and titled ANTIPLAQUE ORAL COMPOSITIONS.

### TECHNICAL FIELD OF THE INVENTION

This disclosure relates generally to the field of pharmacotherapy related to the treatment of disorders related to dental plaque. In particular, the invention relates to antiplaque oral compositions containing a transport enhancer and a chelating agent. More particularly, it relates to such compositions which contain MSM and EDTA.

### BACKGROUND OF THE INVENTION

Dental plaque is responsible for many of the diseases common to the oral cavity including dental caries, periodontitis, gingivitis, and the less common peri-implantitis (similar to periodontitis, but with dental implants).

Dental calculus is an ash gray, yellowish or dark brown calcified substance depositing on dental crowns, exposed dental root surfaces, or surfaces of restorative dental materials. Plaque (dental plaque) adheres to surfaces of human teeth. In the plaque, a reaction occurs by which inorganic salts become more adhesive, and calcification starts from a layer which touches a tooth surface. The calcification advances as the plaque becomes older and thicker, and new plaques adhere to the surface of the calcified plaques and causes calcification. Dental calculus is formed by repetition of this process.

Seventy to eighty percents of components of supragingival dental calculus consist of inorganic salts, and most of them consist of hydroxyapatite (Ca₁₀(PO₄)₆(OH)₂). Calcium phosphate, calcium carbonate, magnesium phosphate and the like may sometimes be also contained. Further, dibasic calcium phosphate and tribasic calcium phosphate may sometimes be contained. Organic components contained in dental calculus are bacterial cells, and they include cell walls of gram positive bacteria and endotoxins as outer membrane components of gram negative bacteria. Dental plaque formation more easily progresses on dental calculus surfaces than on smooth tooth surfaces, and dental plaque adhering to dental calculus stimulates periodontal soft tissues to become etiologic substance. Accordingly, scaling of teeth is important as one of the fundamental treatments in prophylaxis and therapy of periodontal diseases.

For removal of dental calculus, methods involving mechanical removal of calculus by using a scaler or the like have been conventionally applied. However, the methods have problems in that the treatment takes a long period of time due to hardness of dental calculus, and thus patients, dentists, or dental hygienists bear burdens. As a means for chemically dissolving and removing dental calculus agents for dissolving dental calculus are known. However, the dissolution action of the agent is inefficient from a practical point of view. Due to irritation caused by the chemicals on periodontal soft tissues, such agents are not been used in the field of clinical dentistry.

Various oral compositions, such as toothpastes and mouthrinses, that have been manufactured and sold, have primarily based their cleaning abilities on surfactants, soaps, and or detergents along with mild abrasives. Some formulations have added antimicrobial agents, for example TRICLOSAN^{®} or stannous fluoride to enhance antiplaque action.

Therefore it is desired to develop an agent for dissolving dental plaque that can dissolve dental plaque in a short period of time and does not irritate or damage tissues in oral cavity such as periodontal soft tissues or dental tissues.

US 6,645,472 discloses dentifrice combining MSM and ascorbic aicd.

### SUMMARY OF THE INVENTION

MSM and EDTA (chelators in general) were not known to have an anti-plaque effect. Calcium chelators (like EDTA) are expected to have a negative impact on dental enamel and teeth in general. MSM also does not have any anti-plaque properties. However, treatment with a combination of MSM/EDTA surprisingly and unexpectedly showed dramatic reductions in plaque formation.

In some embodiments, the present invention relates to formulations comprising MSM as transport enhancer and a particular chelating agent as defined in claim 1.

In one aspect of the invention, a formulation for use in a process for treating teeth to inhibit plaque development on them as defined in claim 10 is provided.

The process involves administering to the subject an effective amount of a formulation composed of a therapeutically effective amount of a chelating agent as defined in claim 1 and an effective transport-enhancing amount of methylsulfonylmethane (MSM; also referred to as methylsulfone, dimethylsulfone, and DMSO₂).

The formulation may be administered in the form of a paste or gel. Additionally, in a particular embodiment, the formulation is entirely composed of components that are naturally occurring and/or classified as GRAS ("Generally Regarded as Safe") by the U.S. Food and Drug Administration. However, the invention also contemplates non-GRAS components in the formulations.

The invention further provides formulations for use that result in significant reduction of dental plaque.

These and other aspects will become apparent from the following description of the preferred embodiment

### DETAILED DESCRIPTION OF THE INVENTION

The terms used in this specification generally have their ordinary meanings in the art, within the context of the invention, and in the specific context where each term is used. Certain terms that are used to describe the invention are discussed below, or elsewhere in the specification, to provide additional guidance to the practitioner regarding the description of the invention. For convenience, certain terms may be highlighted, for example using italics and/or quotation marks. The use of highlighting has no influence on the scope and meaning of a term; the scope and meaning of a term is the same, in the same context, whether or not it is highlighted. It will be appreciated that same thing can be said in more than one way. Consequently, alternative language and synonyms may be used for any one or more of the terms discussed herein, nor is any special significance to be placed upon whether or not a term is elaborated or discussed herein. Synonyms for certain terms are provided. A recital of one or more synonyms does not exclude the use of other synonyms. The use of examples anywhere in this specification including examples of any terms discussed herein is illustrative only, and in no way limits the scope and meaning of the invention or of any exemplified term. Likewise, the invention is not limited to various embodiments given in this specification.

Throughout this application, various publications, patents and published patent applications are cited. Citation herein of a publication, patent, or published patent application is not an admission the publication, patent, or published patent application is prior art.

As used herein and in the appended claims, the singular forms "a," "and," and "the" include plural referents unless the context clearly dictates otherwise. Thus, for example, "a transport enhancer" encompasses a plurality of transport enhancers as well as a single transport enhancer. Reference to "a chelating agent" includes reference to two or more chelating agents as well as a single chelating agent, and so forth. In this specification and in the claims that follow, reference will be made to a number of terms, which shall be defined to have the following meanings:

The terms "treating" and "treatment" as used herein refer to the administration of an agent or formulation to a clinically symptomatic individual afflicted with an adverse condition, disorder, or disease, so as to effect a reduction in severity and/or frequency of symptoms, eliminate the symptoms and/or their underlying cause, and/or facilitate improvement or remediation of damage. The terms "preventing" and "prevention" refer to the administration of an agent or composition to a clinically asymptomatic individual who is susceptible to a particular adverse condition, disorder, or disease, and thus relates to the prevention of the occurrence of symptoms and/or their underlying cause. Unless otherwise indicated herein, either explicitly or by implication, if the term "treatment" (or "treating") is used without reference to possible prevention, it is intended that prevention be encompassed as well, such that "a method for the treatment of gingivitis" would be interpreted as encompassing "a method for the prevention of gingivitis."

"Optional" or "optionally present" - as in an "optional substituent" or an "optionally present additive" means that the subsequently described component (e.g., substituent or additive) may or may not be present, so that the description includes instances where the component is present and instances where it is not.

By "pharmaceutically acceptable" is meant a material that is not biologically or otherwise undesirable, e.g., the material may be incorporated into a formulation of the invention without causing any undesirable biological effects or interacting in a deleterious manner with any of the other components of the dosage form formulation. However, when the term "pharmaceutically acceptable" is used to refer to a pharmaceutical excipient, it is implied that the excipient has met the required standards of toxicological and manufacturing testing and/or that it is included on the Inactive Ingredient Guide prepared by the U.S. Food and Drug Administration. The terms "treating" and "treatment" as used herein refer to reduction in severity and/or frequency of symptoms, elimination of symptoms and/or underlying cause, prevention of the occurrence of symptoms and/or their underlying cause, and improvement or remediation of an undesirable condition or damage. Thus, for example, "treating" a subject involves prevention of an adverse condition in a susceptible individual as well as treatment of a clinically symptomatic individual by inhibiting or causing regression of the condition. The term "chelating agent" (or "active agent") refers to any chemical compound, complex or composition that exhibits a desirable effect in the biological context, i.e., when administered to a subject or introduced into cells or tissues in vitro. The term includes pharmaceutically acceptable derivatives of those active agents specifically mentioned herein, including, but not limited to, salts, esters, amides, isomers, crystalline forms, and hydrates. When the term "chelating agent" is used, or when a particular chelating agent is specifically identified, it is to be understood that pharmaceutically acceptable salts, esters, amides, and isomers of the agent are intended as well as the agent per se.

By an "effective" amount or a "therapeutically effective" amount of an active agent is meant a nontoxic but sufficient amount of the agent to provide a beneficial effect. The amount of active agent that is "effective" will vary from subject to subject, depending on the age and general condition of the individual, the particular active agent or agents, and the like. Unless otherwise indicated, the term "therapeutically effective" amount as used herein is intended to encompass an amount effective for the prevention of an adverse condition and/or the amelioration of an adverse condition, i.e., in addition to an amount effective for the treatment of an adverse condition.

The term "controlled release" refers to an agent-containing formulation or fraction thereof in which release of the agent is not immediate, i.e., with a "controlled release" formulation, administration does not result in immediate release of the agent into an absorption pool. The term is used interchangeably with "non-immediate release" as defined in Remington: The Science and Practice of pharmacy, Nineteenth Ed. (Easton, Pa.: Mack Publishing Company, 1995). In general, the term "controlled release" as used herein refers to "sustained release" rather than to "delayed release" formulations. The term "sustained release" (synonymous with "extended release") is used in its conventional sense to refer to a formulation that provides for gradual release of an agent over an extended period of time.

An adverse oral condition as that term is used herein may be a "normal" condition that is frequently seen in individuals (e.g., increased dental calculus) or a pathologic condition that may or may not be associated with a named disease. The latter adverse oral conditions include a wide variety of dental disorders and diseases, associated with deposition of mineral deposits, biofilm build-up, infections and inflammation. It should also be emphasized that the present formulation can be advantageously employed to improve oral health, in general, in any mammalian individual.

Unless otherwise indicated, the invention is not limited to specific formulation components, modes of administration, manufacturing processes, or the like, as such may vary.

Unless otherwise defined, all technical and scientific terms used herein have the same meaning as commonly understood by one of ordinary skill in the art to which this invention pertains. In the case of conflict, the present document, including definitions will control.

### Definitions

Chelating agent: Chelation is a chemical combination with a metal in complexes in which the metal is part of a ring. An organic ligand is called a chelator or chelating agent, the chclatc is a metal complex. The larger number of ring closures to a metal atom the more stable is the compound. The stability of a chelate is also related to the number of atoms in the chelate ring. Monodentate ligands which have one coordinating atom like H₂O or NH₃ are easily broken apart by other chemical processes, whereas polydentate chelators, donating multiple binds to metal ion, provide more stable complexes. Chlorophyll, a green plant pigment, is a chelate that consists of a central magnesium atom joined with four complex chelating agent (pyrrole ring). Heme is an iron chelate which contains iron (II) ion in the center of the porphyrin. Chelating agents offers a wide range of sequestrants to control metal ions in aqueous systems. By forming stable water soluble complexes with multivalent metal ions, chelating agents prevent undesired interaction by blocking normal reactivity of metal ions. EDTA (ethylenediamine tetraacetate) is a good example of common chelating agents which have nitrogen atoms and short chain carboxylic groups.

Examples of chelators of iron and calcium include, but are not limited to, Diethylene triamine pentaacetic acid (DTPA), ethylene diamine tetraacetic acid (EDTA), nitrilotriacetic acid (NTA), 1,3-propylene diamine tetraacetic acid (PDTA), Ethylene diamine disuccinic acid (EDDS), and ethylene glycol tetraacetic acid (EGTA).

Compounds useful as chelating agents herein are defined in claim 1.

Suitable biocompatible chelating agents useful in conjunction with the present invention include, without limitation, monomeric polyacids such as EDTA, cyclohexanediamine tetraacetic acid (CDTA), hydroxyethylethylenediamine triacetic acid (HEDTA), diethylenetriamine pentaacetic acid (DTPA), dimercaptopropane sulfonic acid (DMPS), dimercaptosuccinic acid (DMSA), aminotrimethylene phosphonic acid (ATPA), citric acid, pharmaceutically acceptable salts thereof, and combinations of any of the foregoing. Other exemplary chelating agents include: phosphates, e.g., pyrophosphates, tripolyphosphates, and hexametaphosphates.

EDTA and acceptable EDTA salts are particularly preferred, wherein representative acceptable EDTA salts are typically selected from diammonium EDTA, disodium EDTA, dipotassium EDTA, triammonium EDTA, trisodium EDTA, tripotassium EDTA, and calcium disodium EDTA.

EDTA has been widely used as an agent for chelating metals in biological tissue and blood, and has been suggested for inclusion in various formulations. For example, U.S. Pat. No. 6,348,508 to Denick Jr. et al. describes EDTA as a sequestering agent to bind metal ions. In addition to its use as a chelating agent, EDTA has also been widely used as a preservative in place of benzalkonium chloride, as described, for example, in U.S. Pat. No. 6,211,238 to Castillo et al. U.S. Pat. No. 6,265,444 to Bowman et al. discloses use of EDTA as a preservative and stabilizer. However, EDTA has generally not been applied topically in any significant concentration formulations because of its poor penetration across biological membranes and biofilms including skin, cell membranes and even biofilms like dental plaque.

Among the chelating/sequetering materials which may be included in the compositions there may be mentioned biocompatible chelating agents include, without limitation, monomeric polyacids such as EDTA, cyclohexanediamine tetraacetic acid (CDTA), hydroxyethylethylenediamine triacetic acid (HEDTA), diethylenetriamine pentaacetic acid (DTPA), dimercaptopropane sulfonic acid (DMPS), dimercaptosuccinic acid (DMSA), aminotrimethylene phosphonic acid (ATPA), citric acid, pharmaceutically acceptable salts thereof, and combinations of any of the foregoing.

Other exemplary chelating agents include: phosphates, e.g., pyrophosphates, tripolyphosphates, and hexametaphosphates. Other exemplary chelating agents include: phosphates, e.g., pyrophosphates, tripolyphosphates, and hexametaphosphates; chelating antibiotics such as chloroquine and tetracycline; nitrogen-containing chelating agents containing two or more chelating nitrogen atoms within an amino group or in an aromatic ring (e.g., di-amines, 2,2'-bipyridines, etc.); and polyamines such as cyclam (1,4,7,11-tetraazacyclotetradecane), N-(C₁-C₃₀ alkyl)-substituted cyclams (e.g., hexadecyclam, tetramethylhexadecylcyclam), diethylenetriamine (DETA), spermine, diethylnorspermine (DENSPM), diethylhomo-spermine (DEHOP), deferoxamine (N'-{5-[Acetyl(hydroxy)amino]pentyl} -N- [5 -({4- [(5 -aminopentyl)(hydroxy)amino]-4-oxobutanoyl}amino)pentyl]-N-hydroxysuccinamide, or N'-[5-(Acetyl-hydroxy-amino)pentyl]-N-[5-[3-(5 -aminopentyl-hydroxy-carbamoyl) propanoylamino]pentyl] -N-hydroxy-butane diamide); also known as desferrioxamine B, desferoxamine B, DFO-B, DFOA, DFB or desferal), deferiprone, pyridoxal isonicotinoyl hydrazone (PIH), salicylaldehyde isonicotinoyl hydrazone (SIH), ethane-1,2-bis(N-1-amino-3-ethylbutyl-3-thiol).

Additional, suitable biocompatible chelating agents which may be useful for the practice of the current disclosure include EDTA-4-aminoquinoline conjugates such as ([2-(Bis-ethoxycarbonylmethyl-amino)-ethyl]-{[2-(7-chloro-quinolin-4-ylamino)-ethylcarbamoyl]-methyl}-amino)-acetic acid ethyl ester, ([2-(Bis-ethoxycarbonylmethyl-amino)-propyl]-{[2-(7-chloro-quinolin-4-ylamino)-ethylcarbamoyl]-methyl}-amino)-acetic acid ethyl ester, ([3-(Bis-ethoxycarbonylmethyl-amino)-propyl]-{[2-(7-chloro-quinolin-4-ylamino)-ethylcarbamoyl]-methyl}-amino)-acetic acid ethyl ester, ([4-(Bis-ethoxycarbonylmethyl-amino)-butyl]-{[2-(7-chloro-quinolin-4-ylamino)-ethylcarbamoyl]-methyl}-amino)-acetic acid ethyl ester, ([2-(Bis-ethoxymethyl-amino)-ethyl]-{[2-(7-chloro-quinolin-4-ylamino)-ethylcarbamoyl]-methyl}-amino)-acetic acid ethyl ester, ([2-(Bis-ethoxymethyl-amino)-propyl]-{[2-(7-chloro-quinolin-4-ylamino)-ethylcarbamoyl]-methyl}-amino)-acetic acid ethyl ester, ([3-(Bis-ethoxymethyl-amino)-propyl]-{[2-(7-chloro-quinolin-4-ylamino)-ethylcarbamoyl]-methyl}-amino)-acetic acid ethyl ester, ([4-(Bis-ethoxymethyl-amino)-butyl]-{[2-(7-chloro-quinolin-4-ylamino)-ethylcarbamoyl]-methyl}-amino)-acetic acid ethyl ester as described in Solomon et al., Med. Chem. 2: 133-138, 2006.

Additionally, natural chelators including, but not limited to citric acid, phytic acid, lactic acid, acetic acid and their salts may be mentioned. Other natural chelators and weak chelators include but are not limited to curcumin (turmeric), ascorbic acid, succinic acid, and the like.

Described herein are chelating agents which are incorporated in the formulation as a prochelator. A prochelator is any molecule that is converted to a chelator when exposed to the appropriate chemical or physical conditions. For example, BSIH (isonicotinic acid [2-(4,4,5,5-tetramethyl-[1,3,2]dioxaborolan-2-yl)-benzylidene]-hydrazide) prochelators are converted by hydrogen peroxide into SIH (salicylaldehyde isonicotinoyl hydrazone) iron-chelating agents that inhibit iron-catalyzed hydroxyl radical generation.

The inactivated metal ion sequestering agent is sometimes referred to herein as a "prochelator," although sequestration of metal ions can involve sequestration and complexation processes beyond the scope of chelation per se. The term "prochelator" is analogous to the term "prodrug" insofar as a prodrug is a therapeutically inactive agent until activated in vivo, and the prochelator, as well, is incapable of sequestering metal ions until activated in vivo.

Transport Enhancer: The transport enhancer is selected to facilitate the transport of a chelating agent through the tissues, extra-cellular matrices, and/or cell membranes of a body. An "effective amount" of the transport enhancer represents an amount and concentration within a formulation of the invention that is sufficient to provide a measurable increase in the penetration of a chelating agent into one or more of the sites of oral cavity and/or biofilms on oral surfaces in a subject than would otherwise be the case without the inclusion of the transport enhancer within the formulation.

The transport enhancer is present in a formulation of the invention in an amount that ranges from about 0.1 wt.% to 80 wt.% or to about 30 wt.% or more, typically in the range of about 0.1 wt.% to about 20 wt.%, more typically in the range of about 0.5 wt.% to about 11 wt.%, and most typically in the range of about 1.0 wt.% to about 8 wt.%, for instance, 5 wt.%.

The transport enhancer is methylsulfonylmethane (MSM).

### Treatment of Dental Plaque

While studying the impact of chelating agents combined with MSM, we looked at the Loe Sillness dental plaque index on subjects that brushed their teeth with such formulations. A plaque reducing effect superior to TRICLOSAN^{®} containing toothpastes was observed. Further, it was also observed that continued use of the formulation prevented the reformation of the dental plaque to a very large extent compared to a leading "anti-plaque" toothpaste.

Following a single brushing, the MSM/EDTA toothpaste showed 106% greater reduction in plaque compared to a TRICLOSAN^{®} containing toothpaste. This showed a high degree of statistical significance. (See Example 1)

Following two weeks of twice daily brushings, the MSM + chelator results compared to control showed extremely high statistical significance. (See Example 2)

### Formulations

In accordance with the present invention an antiplaque oral composition comprises an orally acceptable vehicle or base for such composition, an effective antiplaque proportion of a chelator or metal sequestrant and methylsulfonylmethane (MSM) for the oral composition in a proportion which produces an unexpected antiplaque action on the teeth of a user of the oral composition.

Neither chelators nor MSM have previously been known as an effective antiplaque compound. Unexpectedly, the two compounds used together reduce plaque formation when the combination is effectively applied to the teeth. EDTA is the highly preferred chelator/sequesterant of this invention. However, other chelators/sequesterents as defined in claim 1 may be employed in replacement of it. Such agents are preferred in oral compositions because multivalent metals are known to increase plaque adhesion to the teeth.

A variety of means can be used to formulate the compositions of the invention. Techniques for formulation and administration may be found in "Remington: The Science and Practice of Pharmacy," Twentieth Edition, Lippincott Williams & Wilkins, Philadelphia, PA (1995). For human or animal administration, preparations should meet sterility, pyrogenicity, and general safety and purity standards comparable to those required by the FDA. Administration of the pharmaceutical formulation can be performed in a variety of ways, as described herein.

The formulation includes an effective amount of MSM as a permeation enhancer.

MSM is an odorless, highly water-soluble (34% w/v @ 79° F.) white crystalline compound with a melting point of 108-110° C. and a molecular weight of 94.1 g/mol. MSM serves as a multifunctional agent herein, insofar as the agent not only increases cell membrane permeability, but also acts as a "transport facilitating agent" (TFA) that aids in the transport of one or more formulation components to oral tissues. MSM additionally possesses unique and beneficial solubilization properties, in that it is soluble in water, as noted above, but exhibits both hydrophilic and hydrophobic properties because of the presence of polar S=O groups and nonpolar methyl groups. The molecular structure of MSM also allows for hydrogen bonding with other molecules, i.e., between the oxygen atom of each S=O group and hydrogen atoms of other molecules, and for formation of van der Waal associations, i.e., between the methyl groups and nonpolar (e.g., hydrocarbyl) segments of other molecules. The concentration of MSM in the present formulations is in the range of about 0.1 wt. % to 80 wt.% or to 40 wt. %, or from about 0.5 wt.% to about 4, 5, 6, 7, 8, 10, 15, 25 wt.%, and preferably between about 1.5 wt. % to 8.0 wt. %.

Other optional additives in the present formulations include secondary enhancers, i.e., one or more additional permeation enhancers. For example, formulation of the invention can contain added DMSO. Since MSM is a metabolite of DMSO (i.e., DMSO is enzymatically converted to MSM), incorporating DMSO into an MSM-containing formulation of the invention will tend to gradually increase the fraction of MSM in the formulation. If DMSO is added as a secondary enhancer, the amount is preferably in the range of about 1.0 wt. % to 2.0 wt. % of the formulation, and the weight ratio of MSM to DMSO is typically in the range of about 1:50 to about 50:1.

The biocompatible chelating agent as defined in claim 1 is a sequestrant of divalent or polyvalent metal cations, and represents about 0.1 wt. % to 40 wt.% or to 15 wt. %, about 0.6 wt. % to 10 wt. %, or preferably about 1.0 wt. % to 5.0 wt. %, of the formulation. Any biocompatible chelating agent can be used in conjunction with the present disclosure providing that it is capable of being buffered to a pH in the range of about 4.5 to about 9.0 and does not interact with any other component of the formulation. Suitable biocompatible chelating agents useful in conjunction with the present invention include, without limitation, monomeric polyacids such as EDTA, cyclohexanediamine tetraacetic acid (CDTA), hydroxyethylethylenediamine triacetic acid (HEDTA), diethylenetriamine pentaacetic acid (DTPA), dimercaptopropane sulfonic acid (DMPS), dimercaptosuccinic acid (DMSA), aminotrimethylene phosphonic acid (ATPA), citric acid, acceptable salts thereof, and combinations of any of the foregoing. Other exemplary chelating agents include: phosphates, e.g., pyrophosphates, tripolyphosphates, and, hexametaphosphates; chelating antibiotics such as chloroquine and tetracycline; nitrogen-containing chelating agents containing two or more chelating nitrogen atoms within an imino group or in an aromatic ring (e.g., diamines, 2,2'-bipyridines, etc.); polyamines such as cyclam (1,4,7,11-tetraazacyclotetradecane), N--(C₁-C₃₀ alkyl)-substituted cyclams (e.g., hexadecyclam, tetramethylhexadecylcycla- m), diethylenetriamine (DETA), spermine, diethylnorspermine (DENSPM), diethylhomo-spermine (DEHOP), and deferoxamine (N'-[5-[[4-[[5-(acetylhydr-oxyamino)pentyl]amino]-1,4-dioxobutyl]hydroxyamino]pentyl]-N'-(5-aminopent- yl)-N-hydroxybutanediamide; also known as desferrioxamine B and DFO); tetrasodium salt of iminodisuccinic acid; salts of poly-asparatic acid; and tetra sodium salts of L-glutamic acid N,N-diacetic acid (GLDA).

The various oral compositions of the invention contain adjuvants and additional active components to make them more acceptable to the consumer and to make them more effective in use.

Among the active materials which may be included in the compositions are azacycloalkane diphosphonic compounds, such as azacycloheptane diphosphonic acid and salts thereof, which have an anticalculus effect. (U.S. Pat. No. 5.096,699). Synthetic anionic polymeric polycarboxylates, such as copolymers of maleic acid or maleic anhydride with vinyl methyl ether, and their salts, e.g., sodium salts, which are sold under the trademark Gantrez^{®}, improve the anticalculus action of the mentioned diphosphonic compounds and also have stabilizing and other desirable effects on other active materials, such as polyphosphates, e.g., sodium pyrophosphate, which are employed as antitartar agents. Fluorides and other sources of fluoride ions, such as sodium fluoride and sodium monofluorophosphate, are also active components which may be utilized, for their tooth hardening effects.

A source of fluoride ions may be water soluble or relatively water insoluble, so long as it releases a sufficient quantity of such ions during use. Among the useful sources of fluoride ions are: soluble alkali metal fluorides, such as sodium and potassium fluorides; copper fluorides, such as cuprous fluoride; tin fluorides, such as stannous fluoride; ammonium fluorosilicate; sodium fluorozirconate; ammonium fluorozirconate; sodium monofluorophosphate; aluminum fluorophosphates (mono-, di- and tri-); and fluorinated sodium calcium pyrophosphate. Of these, alkali metal and tin fluorides, such as sodium and stannous fluorides, sodium monofluorophosphate (MFP^{®}) and mixtures thereof, are preferred.

Polyphosphate anti-tartar agents may include any of various suitable polyphosphates, such as alkali metal tripolyphosphates and pyrophosphates, but sodium pyrophosphate is preferred.

Synthetic anionic polymeric polycarboxylates (SAPP's), which stabilize the polyphosphates and improve the present antiplaque oral compositions too, may be of molecular weights in the range of about 5,000-2,000,000, preferably about 50,000-1,500,000 and more preferably 500,000-1,000,000, e.g., about 1,000,000, and are available from GAF Corporation under the designations Gantrezes^{®} AN-169, AN-139, AN-119 and S-97, pharmaceutical grade. Such SAPP's are all linear copolymers but cross-linked polymers, such as those sold under the trade mark Carbopol^{®}, of B. F. Goodrich, e.g., Carbopols 934, 940 and 941, may be substituted, preferably only in part. Corresponding analogues of the SAPP's may also be substituted, in whole or in part, including known polysulfonates, polysulfates and polyphosphonates. Other olefinic monomers that are copolymerizable with the described acids or anhydrides include vinyl acetates, vinyl chloride, dimethyl maleate and similar unsaturated monomers, and the copolymers made will contain a sufficient proportion of acidic groups or neutralized or neutralizable acidic groups to make them water soluble or swellable. Some such polycarboxylate copolymers are those disclosed in U.S. Pat. Nos. 4,138,477 and 4,183,914, and include copolymers of maleic anhydride with styrene, isobutylene or vinyl ethyl ether, polyacrylic, polyitaconic and polymaleic acids, and sulfoacrylic oligomers of comparatively low molecular weights, such as Uniroyal^{®} ND-2.

Because the compositions are intended for oral uses they can be sweetened with saccharin or aspartame or sucralose or natural sweeteners like STEVIA^{®}. Coloring agents may be employed, as may be speckles or other visual attractants, and in cases where undesirable reactions could occur between components during storage before use, some of such reactants may be separated from others by being incorporated in such speckles or by being packed in dispensing containers having separate sections to prevent such reactions.

Other possible additives for incorporation into the formulations that are at least partially aqueous include, without limitation, thickeners, isotonic agents, buffering agents, and preservatives, providing that any such excipients do not interact in an adverse manner with any of the formulation's other components. It should also be noted that preservatives are not generally necessarily in light of the fact that the selected chelating agent itself serves as a preservative. Suitable thickeners will be known to those of ordinary skill in the art of formulation, and include, by way of example, cellulosic polymers such as methylcellulose (MC), hydroxyethylcellulose (HEC), hydroxypropylcellulose (HPC), hydroxypropylmethylcellulose (HPMC), and sodium carboxymethylcellulose (NaCMC), and other swellable hydrophilic polymers such as polyvinyl alcohol (PVA), hyaluronic acid or a salt thereof (e.g., sodium hyaluronate), and crosslinked acrylic acid polymers commonly referred to as "carbomers" (and available from B.F. Goodrich as Carbopol^{®} polymers). Various organic gums such as but not limited to Xanthan gum and Konjac gum. The preferred amount of any thickener is such that a viscosity above 10,000 cps is provided, as a gel having a viscosity above this figure generally considered optimal for both comfort and retention of the formulation on the oral tissues. Any suitable isotonic agents and buffering agents commonly used in oral formulations may be used, providing the pH of the formulation is maintained in the range of about 4.5 to about 9.0, preferably in the range of about 6.8 to about 7.8, and optimally at a pH of about 7.4.

The formulations of the invention also include a pharmaceutically acceptable carrier, which will depend on the particular type of formulation. The formulations of the invention are provided as a paste or gel, in which case the carrier is at least partially aqueous. Described herein are aslo ointments, in which case the pharmaceutically acceptable carrier is composed of an ointment base. Preferred ointment bases herein have a melting or softening point close to body temperature, and any ointment bases commonly used in oral preparations may be advantageously employed. Common ointment bases include petrolatum and mixtures of petrolatum and mineral oil.

Suitable pharmaceutical formulations and dosage forms may be prepared using conventional methods known to those in the field of pharmaceutical formulation and described in the pertinent texts and literature, e.g., in Remington: The Science and Practice of Pharmacy, 21st edition, Lippincott Williams & Wilkins, 2005.

The chelating agent may be administered, if desired, in the form of a salt, ester, crystalline form, or hydrate, provided it is pharmaceutically acceptable. Salts, esters, etc. may be prepared using standard procedures known to those skilled in the art of synthetic organic chemistry and described, for example, by J. March, Advanced Organic Chemistry: Reactions, Mechanisms and Structure, 4th Ed. (New York: Wiley- Interscience, 1992).

The oral formulations may also include conventional additives such as opacifiers, flavoring agents, antioxidants, fragrance, colorant, gelling agents, thickening agents, stabilizers, surfactants, and the like. Other agents may also be added, such as antimicrobial agents, to prevent spoilage upon storage, i.e., to inhibit growth of microbes such as yeasts and molds. Suitable antimicrobial agents are typically selected from the methyl and propyl esters of p-hydroxybenzoic acid (i.e., methyl and propyl paraben), sodium benzoate, sorbic acid, imidurea, and combinations thereof.

The antiplaque oral compositions of this invention are dentifrices, such as toothpastes and gels, but various other such compositions may also be given the described improved antiplaque properties by including in them a chelating/sequestrating agent as defined in claim 1 and MSM. Such compositions include tooth powders, tooth hardeners, anti-tartar compositions, anti-calculus compositions, gums, tablets and lozenges. For liquid state compositions described herein, such as mouth-rinses, mouthwashes, tooth hardeners and antiplaque and anti-tartar compositions the liquid medium in which the active components are present will normally be aqueous and may be aqueous alcoholic, with ethanol being the preferred alcohol. Such compositions often also contain a humectant, such as a polyol, e.g., glycerol, sorbitol, mannitol, polyethylene glycol, propylene glycol, or a mixture of two or more thereof, and a surfactant, such as a dental detergent or a mixture of such detergents. Other adjuvants and active components may also be present and such will be described later.

For the paste or gel compositions of the invention, such as toothpastes, gel dentifrices, the base or the medium for the active components will usually be any which is employed in such compositions that do not contain the combination of a chelating/sequestrating agent and MSM. For the toothpaste and gel dentifrices such bases will usually comprise: water; humectant; polishing agent, such as finely divided silica, calcium carbonate, tricalcium phosphate, dicalcium phosphate and/or insoluble sodium metaphosphate (of which the finely divided silica polishing agent is preferred); and a surfactant, such as sodium lauryl sulfate, sodium N-coco, N-methyl taurate, sodium N-lauroyl sarcosine, or other compatible dental detergent. A thickener, which will preferably be a natural or synthetic gum, such as carrageenan or hydroxymethyl cellulose, or a siliceous thickener such as fumed silica, or a mixture of such thickeners will also often be employed to help to increase paste or gel viscosity or body and it can function as a gelating agent. Other known thickeners and gelating agents may be employed in place of those specifically mentioned above and other known polishing agents, humectants and surfactants may also be used. Bases for tooth powders will normally be almost entirely of polishing agent, with some surfactant desirably being present. The base for the gum can be an elastomer of a type normally employed in chewing gums, e.g., chicle, gum or rubber, and the tablets and lozenges may have a hard sugar or candy base but preferably will be of sorbitol or a gummy material, such as gelatin.

A chewing gum of the above formula is made by blending together MSM/EDTA with an elastomer (chicle), a humectant (sorbitol/mannitol), a sweetener, a filler (talc), and optionally an anionic polymeric polycarboxylate (gantrez^{®}) in a suitable mixer, such as a Banbury mixer. Such a chewing gum is effective in inhibiting the development of plaque on the teeth when chewed daily, preferably several times daily, for at least seven to ten days. It is also effective when the proportion of the active components, MSM and EDTA are altered while still remaining within the ranges previously given in this specification. For best antiplaque effects the gum should be chewed several times daily for one or more minutes at a time for at least a week.

The treatment regimen will depend on a number of factors that may readily be determined, such as severity of the condition and responsiveness of the condition to be treated, but will normally be one or more treatments per day, with a course of treatment lasting from a day or several days to several months, or until a significant diminution of dental plaque is achieved. Noticeable reduction in dental plaque is observed after about 14 days of twice daily usage.

Typically a significant reduction in dental plaque is at least about 10% reduction as measured by Loe-Sillness dental plaque index following about 4-6 weeks of twice daily brushings. Alternately, significant plaque reduction can be measured as a reduction by about 1.5-fold over control toothpaste as measured by Loe-Sillness dental plaque index following about 4-6 weeks of twice daily brushings. Typical control toothpaste is a standard over-the-counter fluoride containing toothpaste.

When a formulation is provided as a mouth-rinse or mouthwash, exemplary treatment regimens employ rinsings of a duration of at least 30 seconds, at least twice a day, for example in the morning and in late afternoon, for at least 4, or 5, or 7, or 15 days, or a month.

When a formulation is provided as a toothpaste or tooth powder, exemplary treatment regimens employ brushings at least once, at least twice or at least three times a day, for at least 1 day, at least 3 days, at least a week, at least 2 weeks or at least a month.

When a formulation is provided as a lozenge or tablet, exemplary treatment regimens comprise use at least once or twice a day, for at least a week, or 2 weeks or a month, or 2 months.

When a formulation is provided as a chewing gum, exemplary treatment regimens comprise use at least once or twice or several times a day, for at least a week.

### EXAMPLES

The following examples are put forth so as to provide those skilled in the art with a complete invention and description of how to make and use embodiments in accordance with the invention, and are not intended to limit the scope of what the inventors regard as their discovery. Efforts have been made to ensure accuracy with respect to numbers used (e.g. amounts, temperature, etc.) but some experimental errors and deviations should be accounted for. Unless indicated otherwise, parts are parts by weight, molecular weight is weight average molecular weight, temperature is in degrees Centigrade, and pressure is at or near atmospheric.

### Example 1: Reduction of Plaque development after single brushing

Toothpaste was prepared comprising EDTA (tetrasodium salt) and MSM, which were purchased from Sigma. Control was a leading "anti-plaque" toothpaste available in the market. Sodium phytate has been added for comparative purposes and is not according to the invention.

| Treatment | Reduction in Plaque Index |
|---|---|
| Control toothpaste | 1.11 |
| 5.4% MSM / 2.6% EDTA tooth gel | 2.29 |
| 5.0% MSM / 2.0%sodium citrate gel | 1.87 |
| 5.0% MSM / 2.0% sodium phytate gel | 1.73 |

The MSM/EDTA toothpaste showed 106% greater reduction in plaque. This showed a high degree of statistical significance.

### Example 2: Reduction of Plaque development after repeated brushings

Toothpaste was prepared comprising EDTA (tetrasodium salt) and MSM, which were purchased from Sigma. Control was a leading "anti-plaque" toothpaste available in the market. Loe Sillness dental plaque index was measured on subjects after 2 weeks of twice daily brushing, after an initial prophylaxis. Sodium phytate has been added for comparative purposes and is not according to the invention.

| Treatment | Plaque Index |
|---|---|
| Control toothpaste | 1.89 |
| 5.4% MSM / 2.6% EDTA tooth gel | 0.13 |
| 5.0% MSM / 2.0%sodium citrate gel | 0.95 |
| 5.0% MSM / 2.0% sodium phytate gel | 1.18 |

The MSM+chelator results compared to control showed extremely high statistical significance.

## Claims

1. An antiplaque oral formulation, consisting of:
a chelating agent selected from ethylenediamine tetraacetic acid (EDTA), ethylene glycol tetraacetic acid (EGTA), cyclohexanediamine tetraacetic acid (CDTA), hydroxyethylethylenediamine triacetic acid (HEDTA), diethylenetriamine pentaacetic acid (DTPA), dimercaptopropane sulfonic acid (DMPS), dimercaptosuccinic acid (DMSA), aminotrimethylene phosphonic acid (ATPA), citric acid, acetic acid and acceptable salts thereof, and any combinations thereof,
a transport enhancer that is methylsulfonylmethane (MSM);
a thickening agent in an amount sufficient for the formulation to have a viscosity above 10,000 cps;
an orally acceptable vehicle or base for the formulation; and
one or more additives selected from polishing agents, surfactants, humectants, solvents, sweeteners, tooth hardeners, anti-tartar agents, anti-calculus agents, flavoring agents, antibacterial agents, coloring agents, isotonic agents, buffering agents and preservatives,
wherein the chelating agent and the transport enhancer are present in a proportion effective to bring about a significant plaque reduction as measured by the Loe-Sillness index on the teeth of a user of the oral formulation following at least two weeks of twice-daily application,
wherein the percentage of chelator is about 0.1% to 40% and the percentage of transport enhancer in the formulation is about 0.1% to 80% by weight, respectively, and
wherein the oral formulation is in the form of a paste or gel.

2. The formulation of claim 1, wherein the EDTA salt is selected from diammonium EDTA, disodium EDTA, dipotassium EDTA, triammonium EDTA, trisodium EDTA, tripotassium EDTA, calcium disodium EDTA, and combinations thereof.

3. The formulation of claim 1, wherein the polishing agent is selected from one or more of finely divided silica, calcium carbonate, tricalcium phosphate, dicalcium phosphate and insoluble sodium metaphosphate.

4. The formulation of claim 1, wherein the surfactant is selected from one or more of sodium lauryl sulfate, N-methyl taurate, sodium N-lauroyl sarcosine, and a compatible dental detergent.

5. The formulation of claim 1, wherein the thickener is selected from one or more of a natural or synthetic gum, carrageenan, hydroxymethyl cellulose, a siliceous thickener and fumed silica.

6. The formulation of claim 1, wherein the sweetener is selected from one or more of saccharin, aspartame, sucralose, and Stevia.

7. The formulation of claim 1, wherein the anticalculus agent is selected from one or more of an azacycloalkane diphosphonic compound, azacycloheptane diphosphonic acid and salts thereof, synthetic anionic polymeric polycarboxylates, and copolymers of maleic acid or maleic anhydride with vinyl methyl ether, and their salts.

8. The formulation of claim 1, wherein the tooth hardening agent is selected from soluble alkali metal fluorides, sodium fluoride, potassium fluoride, copper fluoride, tin fluorides, ammonium fluorosilicate, sodium fluorozirconate, ammonium fluorozirconate, aluminum fluorophosphates (mono-, di- and tri-), fluorinated sodium calcium pyrophosphate, sodium monofluorophosphate and mixtures thereof.

9. The formulation of claim 1, wherein the anti-tartar agent is selected from polyphosphates, alkali metal tripolyphosphates, alkali metal pyrophosphates and sodium pyrophosphate.

10. A formulation according to claim 1 for use in a process for treating teeth to inhibit plaque development on them, the process comprising:
applying to the teeth a plaque inhibiting amount of said formulation,
wherein a significant decline in dental plaque is measured by the Loe-Sillness index on the teeth following at least two weeks of twice-daily application,
and further wherein:
when the formulation is a tooth paste, the application requires brushing with the formulation at least once a day for at least 3 days,
when the formulation is a mouth-rinse or mouthwash, the application requires rinsing with the formulation at least twice a day for 4 or more days, and
when the formulation is a chewing gum, the application requires use of the formulation at least twice a day for one week or more.

## Patentansprüche

1. Oral anwendbare Formulierung gegen Plaque, die aus Folgendem besteht:
einem Chelatbildner, ausgewählt aus Ethylendiamintetraessigsäure (EDTA), Ethylenglykoltetraessigsäure (EGTA), Cyclohexandiamintetraessigsäure (CDTA), Hydroxyethylethylendiamintriessigsäure (HEDTA), Diethylentriaminpentaessigsäure (DTPA), Dimercaptopropansulfonsäure (DMPS), Dimercaptobernsteinsäure (DMSA), Aminotrimethylenphosphonsäure (ATPA), Zitronensäure, Essigsäure und annehmbaren Salzen davon und jeglichen Kombinationen davon;
einem Transportverstärker, der Methylsulfonylmethan (MSM) ist;
einem Verdickungsmittel in einer Menge, die ausreichend ist, sodass die Formulierung eine Viskosität über 10.000 cps aufweist;
einem oral annehmbaren Träger oder einer oral annehmbaren Basis für die Formulierung; und
einem oder mehreren Zusatzstoffen, ausgewählt aus Poliermitteln, Tensiden, Feuchthaltemitteln, Lösungsmitteln, Süßungsmitteln, Zahnhärtern, Antitartarmitteln, Antikalkulusmitteln, Aromastoffen, antibakteriellen Mitteln, Färbemitteln, isotonischen Mitteln, Puffersubstanzen und Konservierungsstoffen,
wobei der Chelatbildner und der Transportverstärker in einem Anteil vorhanden sind, der wirksam ist, um eine erhebliche Plaque-Reduktion herbeizuführen, gemessen durch den Loe-Silness-Index auf den Zähnen eines Nutzers der oral anwendbaren Formulierung nach mindestens zwei Wochen mit einer Anwendung von zweimal täglich,
wobei der Prozentsatz von Chelator etwa 0,1 % bis 40 % beträgt und der Prozentsatz von Transportverstärker in der Formulierung etwa 0,1 % bis 80 Gew.-% beträgt, und
wobei die oral anwendbare Formulierung in der Form einer Paste oder eines Gels ist.

2. Formulierung nach Anspruch 1, wobei das EDTA-Salz ausgewählt ist aus Diammonium-EDTA, Dinatrium-EDTA, Dikalium-EDTA, Triammonium-EDTA, Trinatrium-EDTA, Trikalium-EDTA, Calciumdinatrium-EDTA und Kombinationen davon.

3. Formulierung nach Anspruch 1, wobei das Poliermittel ausgewählt ist aus einem oder mehreren aus feinteiliger Kieselerde, Calciumcarbonat, Tricalciumphosphat, Dicalciumphosphat und unlöslichem Natriummetaphosphat.

4. Formulierung nach Anspruch 1, wobei das Tensid ausgewählt ist aus einem oder mehreren aus Natriumlaurylsulfat, N-Methyltaurat, Natrium-N-Lauroylsarcosin und einem kompatiblen Zahnreinigungsmittel.

5. Formulierung nach Anspruch 1, wobei das Verdickungsmittel ausgewählt ist aus einem oder mehreren aus einem Pflanzen- oder synthetischen Gummi, Carrageen, Hydroxymethylcellulose, einem silicatischen Verdickungsmittel und pyrogenem Siliciumdioxid.

6. Formulierung nach Anspruch 1, wobei das Süßungsmittel ausgewählt ist aus einem oder mehreren aus Saccharin, Aspartam, Sucralose und Stevia.

7. Formulierung nach Anspruch 1, wobei das Antikalkulusmittel ausgewählt ist aus einem oder mehreren aus einer Azacycloalkan-Diphosphon-Verbindung, Azacycloheptan-Diphosphonsäure und Salzen davon, synthetischen anionischen polymeren Polycarboxylaten und Copolymeren von Maleinsäure oder Maleinsäureanhydrid mit Vinylmethylether und ihren Salzen.

8. Formulierung nach Anspruch 1, wobei der Zahnhärter ausgewählt ist aus löslichen Alkalimetallfluoriden, Natriumfluorid, Kaliumfluorid, Kupferfluorid, Zinnfluoriden, Ammoniumfluorosilicat, Natriumfluorzirkonat, Ammoniumfluorzirkonat, Aluminumfluorphosphaten (Mono-, Di- und Tri-), fluoriertem Natrium-CalciumPyrophosphat, Natrium-Monofluorphosphat und Gemischen davon.

9. Formulierung nach Anspruch 1, wobei das Antitartarmittel ausgewählt ist aus Polyphosphaten, Alkalimetall-Tripolyphosphaten, Alkalimetall-Pyrophosphaten und NatriumPyrophosphat.

10. Formulierung nach Anspruch 1 zur Verwendung in einem Prozess zum Behandeln von Zähnen, um Plaqueentwicklung auf ihnen zu hemmen, wobei der Prozess Folgendes umfasst:
Auftragen auf die Zähne einer Plaque-hemmenden Menge der Formulierung,
wobei ein deutlicher Rückgang von Zahnbelag gemessen wird durch den Loe-Silness-Index auf den Zähnen nach mindestens zwei Wochen mit einer Anwendung von zweimal täglich,
und wobei ferner:
wenn die Formulierung eine Zahncreme ist, die Anwendung das Zähneputzen mit der Formulierung mindestens einmal am Tag mindestens 3 Tage lang erfordert,
wenn die Formulierung eine Mundspülung oder Mundwasser ist, die Anwendung das Spülen mit der Formulierung mindestens zweimal am Tag mindestens 4 Tage lang oder länger erfordert, und
wenn die Formulierung ein Kaugummi ist, die Anwendung die Verwendung der Formulierung mindestens zweimal am Tag eine Woche lang oder länger erfordert.

## Revendications

1. Formulation orale antiplaque, constituée par :
un agent chélatant choisi parmi l'acide tétraacétique d'éthylènediamine (EDTA), l'acide tétraacétique d'éthylène glycol (EGTA), l'acide tétraacétique de cyclohexanediamine (CDTA), acide triacétique d'hydroxyéthyléthylènediamine (HEDTA), l'acide pentaacétique de diéthylènetriamine (DTPA), l'acide sulfonique de dimercaptopropane (DMPS), l'acide dimercaptosuccinique (DMSA), l'acide phosphonique d'aminotriméthylène (ATPA), l'acide citrique, l'acide acétique et les sels acceptables de ceux-ci, et toute combinaison de ceux-ci ;
un activateur de transport qui est le méthylsulfonylméthane (MSM);
un agent épaississant en quantité suffisante pour que la formulation ait une viscosité supérieure à 10000 cps ;
un véhicule ou une base oralement acceptable pour la formulation ; et
un ou plusieurs additifs choisis parmi les agents de polissage, les tensioactifs, les humectants, les solvants, les édulcorants, les durcisseurs de dents, les agents antitartre, les agents anti-calcul, les agents aromatisants, les agents antibactériens, les agents colorants, les agents isotoniques, les agents tampons et les conservateurs,
dans laquelle l'agent chélatant et l'activateur de transport sont présents dans une proportion efficace pour provoquer une réduction significative de la plaque dentaire telle que mesurée par l'indice de Loe-Sillness sur les dents d'un utilisateur de la formulation orale après au moins deux semaines d'application biquotidienne,
dans laquelle le pourcentage de chélateur est d'environ 0,1 % à 40 % et le pourcentage d'activateur de transport dans la formulation est d'environ 0,1 % à 80 % en poids, respectivement, et
dans laquelle la formulation orale est sous la forme d'une pâte ou d'un gel.

2. Formulation selon la revendication 1, dans laquelle le sel d'EDTA est choisi parmi l'EDTA de diammonium, l'EDTA de disodium, l'EDTA de dipotassium, l'EDTA de triammonium, l'EDTA de trisodium, l'EDTA de tripotassium, l'EDTA de disodium de calcium et les combinaisons de ceux-ci.

3. Formulation selon la revendication 1, dans laquelle l'agent de polissage est choisi parmi un ou plusieurs parmi la silice finement divisée, le carbonate de calcium, le phosphate tricalcique, le phosphate dicalcique et le métaphosphate de sodium insoluble.

4. Formulation selon la revendication 1, dans laquelle le tensioactif est choisi parmi un ou plusieurs parmi le sodium laurylsulfate, N-méthyltaurate, sodium N-lauroyl sarcosine et un détergent dentaire compatible.

5. Formulation selon la revendication 1, dans laquelle l'épaississant est choisi parmi un ou plusieurs parmi une gomme naturelle ou synthétique, le carraghénane, l'hydroxyméthylcellulose, un épaississant siliceux et la silice fumée.

6. Formulation selon la revendication 1, dans laquelle l'édulcorant est choisi parmi un ou plusieurs parmi la saccharine, l'aspartame, le sucralose et la stévia.

7. Formulation selon la revendication 1, dans laquelle l'agent antitartre est choisi parmi un ou plusieurs parmi un composé diphosphonique d'azacycloalcane, l'acide diphosphonique d'azacycloheptane et les sels de ceux-ci, les polycarboxylates polymères anioniques synthétiques et les copolymères d' acide maléique ou d'anhydride maléique avec l'éther vinyl méthylique, et les sels de ceux-ci.

8. Formulation selon la revendication 1, dans laquelle l'agent de durcissement dentaire est choisi parmi les fluorures de métaux alcalins solubles, le fluorure de sodium, le fluorure de potassium, le fluorure de cuivre, les fluondes d'étain, le fluorosilicate d'ammonium, le fluorozirconate de sodium, le fluorozirconate d'ammonium, les fluorophosphates d'aluminium (mono, di et tri), le pyrophosphate de calcium sodique fluoré, le monofluorophosphate de sodium et les mélanges de ceux-ci.

9. Formulation selon la revendication 1, dans laquelle l'agent antitartre est choisi parmi les polyphosphates, les tripolyphosphates de métaux alcalins, les pyrophosphates de métaux alcalins et le pyrophosphate de sodium.

10. Formulation selon la revendication 1 pour une utilisation dans un procédé de traitement des dents pour inhiber le développement de la plaque sur celles-ci, le procédé comprenant :
l'application sur les dents d'une quantité inhibitrice de plaque de ladite formulation,
dans laquelle une diminution significative de la plaque dentaire est mesurée par l'indice de Loe-Sillness sur les dents après au moins deux semaines d'application biquotidienne,
et en outre dans laquelle :
lorsque la formulation est un dentifrice, l'application nécessite un brossage avec la formulation au moins une fois par jour pendant au moins 3 jours,
lorsque la formulation est un rince-bouche ou un bain de bouche, l'application nécessite un rinçage avec la formulation au moins deux fois par jour pendant 4 jours ou plus, et
lorsque la formulation est une gomme à mâcher, l'application nécessite l'utilisation de la formulation au moins deux fois par jour pendant une semaine ou plus.
